(19) Europäisches Patentamt / European Patent Office / Office européen des brevets

(11) **EP 4 353 218 A1**

(12) **EUROPEAN PATENT APPLICATION**
published in accordance with Art. 153(4) EPC

(43) Date of publication:
**17.04.2024 Bulletin 2024/16**

(51) International Patent Classification (IPC):
**A61K 8/19** (2006.01)     **A61Q 1/02** (2006.01)

(21) Application number: **22804388.1**

(52) Cooperative Patent Classification (CPC):
**A61K 8/19; A61Q 1/02**

(22) Date of filing: **25.03.2022**

(86) International application number:
**PCT/JP2022/014359**

(87) International publication number:
**WO 2022/244470 (24.11.2022 Gazette 2022/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.05.2021 JP 2021084057**

(71) Applicant: **Titan Kogyo Kabushiki Kaisha**
**Ube-shi**
**Yamaguchi 755-8567 (JP)**

(72) Inventors:
• **YOSHIMI, Akira**
**Ube-shi, Yamaguchi 755-8567 (JP)**
• **TAKADA, Akihiko**
**Ube-shi, Yamaguchi 755-8567 (JP)**

(74) Representative: **Cabinet Beau de Loménie**
**158, rue de l'Université**
**75340 Paris Cedex 07 (FR)**

(54) **IRON OXIDE PIGMENT FOR COSMETIC COMPOSITION AND COSMETIC COMPOSITION CONTAINING IRON OXIDE PIGMENT**

(57) Provided is an iron oxide pigment that is for compounding with a cosmetic composition, exhibits a large a value and/or value and a high chroma even in areas with low luminance, and has high coloring power. The iron oxide pigment for a cosmetic composition comprises a spinel structure or a spinel structure and a corundum structure, has an elemental Fe content of 680-702 g/kg, an FeO content of 30 g/kg or less, an $Fe_2O_3$ content of 939 g/kg or more, and a total content of 968 g/kg or more of FeO and $Fe_2O_3$.

Fig. 4

EP 4 353 218 A1

**Description**

TECHNICAL FIELD

The present invention relates to an iron oxide pigment for a cosmetic composition and a cosmetic composition containing an iron oxide pigment. The iron oxide pigment of the present invention is particularly suitable for use mainly in skin cosmetic compositions oriented to dark color.

BACKGROUND ART

At the moment, huge changes occur in environments relating to cosmetics. Formerly, people, who were rich enough to purchase industrially-produced cosmetics, lived in limited countries and districts, and cosmetics in demand on the market were those suitable for the physical characteristics or sense of values of the main members of such countries and districts. However, in recent years, due to changes in the global economy or the population structure of each country, countries and districts where rich people live have broadened more than ever, and new and diverse cosmetics have been in demand. In the field of skin cosmetics, a huge need for cosmetics oriented to skin with a small lightness, so-called dark-color skin, which has not been thus far valued, is being created.

Pigments containing iron oxide as a main component have various colors, such as red, yellow, and black, depending on oxidation or reduction, and an excellent coloring power. Further, harmfulness of the pigments to human bodies is small, and thus the pigments are widely used for cosmetics, even in the field of skin cosmetics. An example of Japanese Patent Laid-Open No. 2014-101298 (Patent Literature 1) describes that an aqueous dispersion for a brown cosmetic is manufactured by appropriately combining carbon black or the like with red iron oxide or yellow iron oxide, and that a pen-type eyeliner is produced using the same dispersion. Patent Literature 1 describes that the use of specific iron oxide fine particles as the iron oxide prevents the separation or precipitation of a pigment over time and the clogging of a pen-type container. However, as in Patent Literature 1, in a case where brown color has been produced by mixing pigments having various colors , the components are normally separated from each other due to a difference in the physical properties of each pigment during storage (hereinafter, this phenomenon will be referred to as "color separation"), and the use as a cosmetic is likely to become difficult. In addition, there is no description regarding the coloring power of the pigments in Patent Literature 1.

Japanese Patent Laid-Open No. H02-145506 (Patent Literature 2) describes a brown liquid cosmetic comprising at least water and black iron oxide oxidized with an oxidant, and describes that color separation can be prevented by using the black iron oxide that was oxidized to be brown as a colorant of the liquid. Patent Literature 2 describes that this brown liquid cosmetic is used as eyeliners or the like.

Incidentally, in a case where the brown liquid cosmetic is used as skin cosmetics, there is a demand for precise adjustment of the color compared with the case of being used as eyeliners. Skin cosmetics need to produce a vivid and healthy appearance. On the other hand, in a case where there is a huge difference in color from surrounding skin, skin cosmetics sometimes impart an unnatural impression due to the reflection of light. In conventional cosmetics, the color has been adjusted by blending a tone modifying agent. In a case where a desired color was in a region with a large lightness, it was possible to adjust the color by blending a tone modifying agent. However, in a region with a small lightness such as dark-color skin, since the chroma was also small, it was difficult to adjust a subtle difference in color by blending a tone modifying agent, and it was also difficult to produce vividness.

In addition, since skin cosmetics are applied to broad ranges, there is a strong demand for texture or usability. Here, in a case where an iron oxide pigment has a small coloring power, it is necessary to blend a large amount of the pigment into cosmetic compositions, consumers feel a powdery texture when using cosmetics, and it also becomes difficult to apply cosmetics to broad ranges.

Skin cosmetics oriented to dark color have large chroma even in regions with a small lightness, and it is desirable that a pigment to be contained therein have a large coloring power. The brown liquid cosmetic of Literature 2 does not mention chroma and the coloring power of pigments in regions with a small lightness.

CITATION LIST

PATENT LITERATURE

PTL 1: Japanese Patent Laid-Open No. 2014-101298
PTL 2: Japanese Patent Laid-Open No. H02-145506

## SUMMARY OF INVENTION

### TECHNICAL PROBLEM

The present invention provides an iron oxide pigment for a cosmetic composition exhibiting large chroma even in a region with a small lightness and having a large coloring power. Iron oxide pigments having characteristics as described above are suitable for use particularly in skin cosmetic compositions oriented to dark color.

### SOLUTION TO PROBLEM

As a result of intensive studies with attention paid to a composition of an iron oxide pigment that is intended to be blended into cosmetics, the present inventors have found that an iron oxide pigment having a spinel structure or having a spinel structure and a corundum structure, in which the content of a Fe element is 680 g/kg or more and 702 g/kg or less, the FeO content is 30 g/kg or less, the $Fe_2O_3$ content is 939 g/kg or more, and the total content of FeO and $Fe_2O_3$ is 968 g/kg or more, exhibits large chroma even in a region with a small lightness and, furthermore, has a large coloring power of pigments when blended into cosmetic compositions.

The iron oxide pigment for a cosmetic composition and the cosmetic composition of the present invention are not limited, but include the followings.

[1] An iron oxide pigment for a cosmetic composition, wherein the iron oxide has a spinel structure or has a spinel structure and a corundum structure, and wherein a content of a Fe element is 680 g/kg or more and 702 g/kg or less, a FeO content is 30 g/kg or less, a $Fe_2O_3$ content is 939 g/kg or more, and a total content of FeO and $Fe_2O_3$ is 968 g/kg or more.

[2] The iron oxide pigment according to [1], wherein Pb is 10 mg/kg or less, As is 3 mg/kg or less, Hg is 1 mg/kg or less, Cd is 1 mg/kg or less, Zn is 100 mg/kg or less, Ba is 50 mg/kg or less, Cr is 100 mg/kg or less, Cu is 50 mg/kg or less, and Ni is 200 mg/kg or less.

[3] The iron oxide pigment according to [1] or [2], wherein, when an integrated intensity of diffraction lines of a (311) plane of a spinel-structured iron oxide appearing at a diffraction angle in a range of 35.10° or more and 36.10° or less in X-ray diffraction measurement is regarded as 100.00, an integrated intensity of diffraction lines of a (104) plane of a corundum-structured iron oxide appearing at a diffraction angle in a range of 32.60° or more and 33.60° or less is 10.0 or less.

[4] The iron oxide pigment according to any one of [1] to [3], wherein an average short axis length of primary particles is 50 nm or more and 500 nm or less.

[5] The iron oxide pigment according to any one of [1] to [4], wherein a BET specific surface area is 2.0 $m^2$/g or more and 25.0 $m^2$/g or less, a bulk density is 70 g/mL or less, and a residue after the iron oxide pigment is passed through a sieve with a mesh size of 45 $\mu$m is 1.0 g/kg or less.

[6] The iron oxide pigment according to any one of [1] to [5], wherein oil absorption is 20 g/100 g or more and 50 g/100 g or less.

[7] A cosmetic composition containing the iron oxide pigment according to any one of [1] to [6].

[8] The cosmetic composition according to [7] that is a skin cosmetic.

[9] The cosmetic composition according to [7] or [8] that is a liquid cosmetic.

[10] The cosmetic composition according to [7] or [8] that is a solid cosmetic.

[11] The cosmetic composition according to [7] or [8] that is a gel cosmetic.

[12] The cosmetic composition according to any one of [7] to [11] that is used to produce dark color.

### EFFECTS OF INVENTION

The iron oxide pigment of the present invention exhibits a large a value and/or a large b value and large chroma even in a region with a small lightness when blended into cosmetic compositions. In addition, the iron oxide pigment has a large coloring power. From these characteristics, particularly, the iron oxide pigment can be said to be suitable for use as a colorant for skin cosmetics oriented to dark-color skin. In addition, when blended into cosmetic compositions, the iron oxide pigment of the present invention has a smooth texture and good usability that allows easy application to wide ranges.

In addition, a cosmetic composition obtained by blending the iron oxide pigment of the present invention has characteristics of being easy to manufacture, having a small risk on users' health, and the like.

BRIEF DESCRIPTION OF DRAWINGS

[Fig. 1] Fig. 1 shows the X-ray diffraction measurement results of iron oxide pigments for blending into a cosmetic composition of the present invention.
[Fig. 2] Fig. 2 shows the relationships between the lightness and a value of cosmetic compositions obtained according to the present invention.
[Fig. 3] Fig. 3 shows the relationships between the lightness and b value of the cosmetic compositions obtained according to the present invention.
[Fig. 4] Fig. 4 shows the relationships between the lightness and chroma of the cosmetic compositions obtained according to the present invention.

DESCRIPTION OF EMBODIMENT

In an iron oxide pigment of the present invention for being blended into a cosmetic composition, the content of a Fe element needs to be 680 g/kg or more and 702 g/kg or less. Furthermore, the content of FeO is 30 g/kg or less, the content of $Fe_2O_3$ is 939 g/kg or more, and the total content of FeO and $Fe_2O_3$ is 968 g/kg or more. In the present invention, the contents of the Fe element, FeO, and $Fe_2O_3$ need to be within the above-described ranges in order to exhibit a brown color with large chroma. In a case where the content of FeO is more than 30 g/kg or the content of the element Fe is more than 702 g/kg, the cosmetic composition having the pigment becomes a blackened color with small chroma. In addition, in a case where, in the pigment, the content of the Fe element is less than 680 g/kg or the content of $Fe_2O_3$ is less than 939 g/kg, or the total content of FeO and $Fe_2O_3$ is less than 962 g/kg, a cosmetic composition to be obtained has a dull shade, and the chroma becomes small. More preferably, the content of the Fe element is 680 g/kg or more and 701 g/kg or less, the FeO content is 20 g/kg or less, the $Fe_2O_3$ content is 950 g/kg or more, and the total content of FeO and $Fe_2O_3$ is 970 g/kg or more.

The iron oxide pigment of the present invention for being blended into a cosmetic composition desirably has a spinel structure or has a spinel structure and a corundum structure. In a case where the iron oxide pigment has a spinel structure and a corundum structure, the intensity of diffraction lines derived from the corundum structure is desirably small. Specifically, it is desirable that, when the integrated intensity of diffraction lines of a (311) plane of a spinel-structured iron oxide appearing at a diffraction angle in a range of 35.10° or more and 36.10° or less in X-ray diffraction measurement is regarded as 100.00, the integrated intensity of diffraction lines of a (104) plane of a corundum-structured iron oxide appearing at a diffraction angle in a range of 32.60° or more and 33.60° or less is 10.0 or less. In a case where the diffraction line intensity of the corundum structure is large, the lightness of a cosmetic composition to be obtained becomes large, and it becomes difficult to realize a desired color. The integrated intensity of the diffraction lines derived from the corundum structure is more preferably 7.0 or less and still more preferably 5.0 or less.

In the iron oxide pigment of the present invention for being blended into a cosmetic composition, the contents of heavy metals are desirably small. Specifically, it is desirable that Pb contained in the pigment is 10 mg/kg or less, As is 3 mg/kg or less, Hg is 1 mg/kg or less, Cd is 1 mg/kg or less, Zn is 100 mg/kg or less, Ba is 50 mg/kg or less, Cr is 100 mg/kg or less, Cu is 50 mg/kg or less, and Ni is 200 mg/kg or less. Decrease in the contents of the oxides or salts of the heavy metals leads to suppression of light scattering and absorption due to the oxides or salts, and a decrease in the chroma can be suppressed. In addition, the contents of the heavy metals are desirably small from the viewpoint of use as cosmetics. Particularly, in recent years, consumers set importance to safety of cosmetics, and the regulations on heavy metal contents have been tightened in many countries. A decrease in the heavy metal contents makes it possible to avoid a risk of the revision of sales plans in response to tightened regulations or a risk of the consumer complaint. Regarding the contents of the heavy metals, it is more preferable that Pb is 2 mg/kg or less, As is 2 mg/kg or less, Hg is 1 mg/kg or less, Cd is 1 mg/kg or less, Zn is 20 mg/kg or less, Ba is 5 mg/kg or less, Cr is 20 mg/kg or less, Cu is 10 mg/kg or less, and Ni is 5 mg/kg or less.

The iron oxide pigment of the present invention for being blended into a cosmetic composition, the average short axis length of primary particles is desirably 50 nm or more and 500 nm or less. When the average short axis length is 50 nm or more, the chroma is likely to become large, and, when the average short axis length is 500 nm or less, the coloring power is likely to become large. The average short axis length is more preferably 100 nm or more and 350 nm or less and still more preferably 100 nm or more and 300 nm or less. The long axis lengths are not limited. The ratio between the average long axis length to the average short axis length is not particularly limited, but iron oxide pigments having a ratio of approximately 1 to 200 are ordinarily used.

The average short axis length and the average long axis length of the primary particles of the iron oxide pigment can be determined by, for example, observing approximately 100 to 500 primary particles of the pigment using a transmission electron microscope and calculating the average value thereof.

In the iron oxide pigment of the present invention for being blended into a cosmetic composition, the specific surface area measured by the BET method (hereinafter, referred to as "BET specific surface area") is desirably 2.0 m$^2$/g or more and 25.0 m$^2$/g or less. When the BET specific surface area is 2.0 m$^2$/g or more, the coloring power is likely to become large, and, when the BET specific surface area is 25.0 m$^2$/g or less, the generation of aggregates is suppressed, and a uniform texture is likely to be obtained. The BET specific surface area is more preferably 3.0 m$^2$/g or more and 20.0 m$^2$/g or less, still more preferably 4.0 m$^2$/g or more and 15.0 m$^2$/g or less, and far still more preferably 5.0 m$^2$/g or more and 14.0 m$^2$/g or less.

In the iron oxide pigment of the present invention for being blended into a cosmetic composition, the bulk density is desirably 70 g/mL or less. When the bulk density is 70 g/mL or less, the chroma is likely to become large. The bulk density is preferably 10 g/mL or more and 67 g/mL or less, more preferably 10 g/mL or more and 65 g/mL or less, and still more preferably 15 g/mL or more and 45 g/mL or less.

The bulk density can be measured by, for example, the method according to JIS K 5101-12-1 as described in the section of examples.

In the iron oxide pigment of the present invention for being blended into a cosmetic composition, the residue after the iron oxide pigment is passed through a sieve with a mesh size of 45 μm is desirably 1.0 g/kg or less. When the residue on sieve is 1.0 g/kg or less, the number of clumps of the pigment is small, and it becomes easy to reduce a scratchy unpleasant sensation at the time of using cosmetic compositions. The residue on sieve is more preferably 0.5 g/kg or less and still more preferably 0.3 g/kg or less.

The residue on sieve can be measured by, for example, the method according to JIS K 5101-14-1 as described in the section of EXAMPLES below.

In the iron oxide pigment of the present invention for being blended into a cosmetic composition, the oil absorption is desirably 20 g/100 g or more and 50 g/100 g or less. When the oil absorption is 50 g/100 g or less, the oiliness of cosmetic compositions is reduced, and it becomes easy to reduce an unpleasant sensation attributed to oiliness at the time of using cosmetic compositions. In addition, when the oil absorption is 20 g/100 g or more, it is possible to suppress makeup-comes-off due to sebum. The oil absorption is more preferably 25 g/100 g or more and 45 g/100 g or less.

The oil absorption can be measured by, for example, the method according to JIS K 5101-13-2 as described in the section of EXAMPLES below.

A method for manufacturing the iron oxide pigment of the present invention for being blended into a cosmetic composition is not particularly limited. Ordinarily, the iron oxide pigment can be manufactured by a method in which yellow iron oxide is dehydrated, reduced, and further oxidized or a method in which black iron oxide is oxidized.

Here, the yellow iron oxide is composed of an iron oxide called goethite, which is ordinarily represented by a chemical formula α-FeOOH. The yellow iron oxide ordinarily exhibits yellow color according to JIS custom color name or a color close to yellow, which may vary with observers or environments. The black iron oxide is composed of an iron oxide called magnetite, which is ordinarily represented by a chemical formula $Fe_3O_4$. The black iron oxide ordinarily exhibits a color close to black according to JIS custom color name, which may vary with observers or environments.

The yellow iron oxide or the black iron oxide can be synthesized using a known technique. As an example, the yellow iron oxide can be manufactured by holding a divalent iron salt aqueous solution at a constant temperature within a range from 20°C to 65°C to be neutralized with an alkali, oxidizing the iron with an oxygen-containing gas, then, stirring and aging, and then mixing this and a divalent iron salt aqueous solution, adding an alkali thereto to neutralize the mixture, oxidizing the mixture with an oxygen-containing gas, filtering and drying the mixture. In addition, the black iron oxide can be manufactured by generating a ferrous hydroxide precipitate by a neutralization reaction between a divalent iron salt aqueous solution and an alkali hydroxide, and oxidizing the ferrous hydroxide precipitate by blowing an air thereto while managing the pH and temperature of the aqueous solution. In these manufacturing methods, the composition or particle diameters of iron oxide particles to be obtained can be controlled by controlling the pH of the aqueous solution or the oxidation time.

In the case of obtaining the iron oxide pigment of the present invention for being blended into a cosmetic composition from the yellow iron oxide, the iron oxide pigment can be obtained by dehydrating and reducing the yellow iron oxide and further oxidizing it. An example of the method includes dehydrating the yellow iron oxide, reducing the dehydrated product, and then oxidizing the dehydrated and reduced product. For example, while not limited thereto, an iron oxide pigment suitable for the present invention can be obtained by a method including firing the yellow iron oxide at 200°C in the air, firing the product at 300°C in a hydrogen atmosphere, and, furthermore, firing the product at 200°C in the air. A kiln is generally used for all firing, and it is desirable to perform firing while rotating the kiln. In addition, the reduction is not limited to the hydrogen atmosphere, and an organic substance may be used or other reducing gases may be used.

In the case of obtaining the iron oxide pigment of the present invention for being blended into a cosmetic composition from the black iron oxide, the iron oxide pigment can be obtained by oxidizing the black iron oxide. Specifically, an iron oxide pigment suitable for the present invention can be obtained by firing the black iron oxide in the air. The number of times or duration of the firing is not limited, and the black iron oxide may be fired a plurality of times. In addition, at that time, the temperature may be changed.

In addition, oxidization of the black iron oxide may be performed in water. Specifically, the iron oxide pigment can be obtained by dispersing black iron oxide particles in water and then bringing the particles into contact with an air or adding an oxidant. A method for bringing the particles into contact with an air is not particularly limited, and ordinary, an air is blown into a reaction tank. The oxidant is not particularly limited, and, as an example, hydrogen peroxide or the like can be suitably used. The liquid temperature is not limited; however, ordinarily, 60°C or higher is industrially preferable since the oxidation rate becomes fast, and 90°C or lower is also preferable from the viewpoint of safety since boiling does not occur.

The iron oxide pigment of the present invention for being blended into a cosmetic composition may be obtained using methods other than the above-described methods. For example, the iron oxide pigment for a cosmetic composition of the present invention can be manufactured with reference to a method that is described in Japanese Patent Laid-Open No. H11-092148 in which an iron material is heated and oxidized and then iron oxide is peeled off from the iron material to separate and collect maghemite ($\gamma$-Fe$_2$O$_3$). In addition, the iron oxide pigment for a cosmetic composition of the present invention can be manufactured with reference to a method that is described in Japanese Patent Laid-Open No. 2000-336496 in which a voltage is applied to an electrolytic solution containing an iron ion, a precipitated substance is oxidized and heated to obtain maghemite ultrafine particles. However, for manufacturing methods in which neither the yellow iron oxide nor the black iron oxide is used, ordinarily, a special device or environment is required, and the cost tends to rise.

For the iron oxide pigment of the present invention for being blended into a cosmetic composition, a coating layer of an inorganic substance, such as a hydrous oxide or oxide of a metal such as aluminum, silicon, zinc, titanium, zirconium, iron, cerium, or tin, may be provided on at least a part of the surface of the pigment particle for the purpose of improvement in dispersion stability in dispersion media and durability, for example, at the time of manufacturing cosmetic compositions. In addition, metal salts other than what has described above may also be used as the coating of the inorganic substance. In addition, on at least a part of the particle surface, a coating layer of an organic substance may be provided to perform surface modification represented by a hydrophobilization treatment. As the coating of the organic substance, a silicone compound such as dimethyl polysiloxane or methyl hydrogen polysiloxane, a silane-based, aluminum-based, titanium-based, zirconium-based or other coupling agent, a fluorine compound such as a perfluoroalkyl phosphate compound, a hydrocarbon, lecithin, an amino acid, polyethylene, wax, a metal soap or the like can be treated. A plurality of these treatments may be performed in combination, and, at that time, the order of the treatments is not particularly limited.

The iron oxide pigment of the present invention for being blended into a cosmetic composition is preferably washed or purified after being manufactured within the scope of the common sense of persons who manufacture materials for being blended into cosmetic compositions. When an impurity remains in the iron oxide pigment, there is a possibility that a function as a cosmetic may be affected.

A cosmetic composition of the present invention can be manufactured by combining an iron oxide pigment having the above-described characteristics with other materials necessary for the cosmetic composition. A method for manufacturing the cosmetic composition is not particularly limited. For example, the cosmetic composition can be manufactured by mixing an iron oxide pigment with other components and providing a desired form such as a liquid form, a solid form, or a gel form as described below. For example, in the case of producing a solid cosmetic composition, it is possible to perform drying, molding or the like as necessary after the mixing of each component. Devices that are used for mixing, drying, molding, or the like are not particularly limited.

The cosmetic composition of the present invention desirably contains 0.1 g/kg or more and 500 g/kg or less of the iron oxide pigment of the present invention having the above-described characteristics. When the amount of the iron oxide pigment blended is 500 g/kg or less, it is possible to prevent the cosmetic composition from having a powdery texture, and it becomes easy to apply the cosmetic composition in a wide range. The amount of the iron oxide pigment blended is more preferably 0.1 g/kg or more and 450 g/kg or less, more preferably 0.1 g/kg or more and 400 g/kg or less, and still more preferably 0.1 g/kg or more and 300.0 g/kg or less.

In the cosmetic composition of the present invention, only the iron oxide pigment of the present invention may be used as a colorant, or the color may be adjusted by adding other pigments to a quantitative and qualitative extent that the effect of the present invention is not impaired. As an inorganic pigment that can be jointly used normally, it is possible to use titanium oxide, zinc oxide, red iron oxide, yellow iron oxide, black iron oxide, ultramarine, Prussian blue, red iron oxide, cerium oxide, talc, muscovite, synthetic mica, phlogopite, biotite, synthetic fluorine mica, titanium mica, mica-like iron oxide, sericite, zeolite, kaolin, bentonite, clay, silicic acid, silicic anhydride, magnesium silicate, magnesium aluminum silicate, calcium silicate, barium sulfate, magnesium sulfate, calcium sulfate, calcium carbonate, magnesium carbonate, boron nitride, bismuth oxychloride, alumina, zirconium oxide, magnesium oxide, chromium oxide, calamine, hydroxyapatite, a complex thereof, or the like. As an organic pigment that can be jointly used in the same manner, it is possible to use a silicone powder, a silicone elastic powder, a polyurethane powder, a cellulose powder, a nylon powder, a urethane powder, a silk powder, a PMMA powder, starch, a polyethylene powder, a polystyrene powder, carbon black, a tar dye, a natural dye, a metallic soap such as zinc stearate, or the like, a complex thereof, or the like. In a case where a pigment other than the iron oxide pigment of the present invention is added to the cosmetic composition of the present invention, as the pigment that is added, among them, red iron oxide, yellow iron oxide and/or black iron oxide are preferable from the viewpoint of preventing color separation.

The red iron oxide is composed of an iron oxide called hematite, which is ordinarily represented by a chemical formula $\alpha$-$Fe_2O_3$. The red iron oxide ordinarily exhibits a color close to red according to JIS custom color name or red, which may vary with observers or environments.

Into the cosmetic composition containing the iron oxide pigment of the present invention, a component other than pigments can be blended to a quantitative and qualitative extent that the effect of the present invention is not impaired depending on the purpose. For example, one or more of an oily component, a dye, a pH adjuster, a moisturizer, a thickener, a surfactant, a dispersant, a stabilizer, a preservative, an antioxidant, a sequestering agent, an astringent, an antiphlogistic agent, a UV absorber, a perfume, an abrasive, and the like can be blended as appropriate. Particularly, when a plate-like compound represented by mica is blended, the slipperiness of the cosmetic composition becomes favorable, which is desirable.

(Uses of Cosmetics)

The cosmetic composition containing the iron oxide pigment of the present invention exhibits a large a value and/or a large b value and large chroma even in a region with a small lightness, furthermore, has a large coloring power of the pigment, and is thus suitable for use particularly as skin cosmetics oriented to dark color. The cosmetic composition of the present invention is capable of imparting a natural impression while producing a healthy appearance in a vivid color even in the case of being used as skin cosmetics oriented to dark color. In the present specification, "vivid" is the same meaning as "large chroma." In addition, the cosmetic composition is capable of producing dark color, which, conventionally, could not be produced without mixing two or more kinds of iron oxide pigments, with a single pigment and, in that case, can be used as skin cosmetics not causing color separation. In addition, even in the case of producing dark color by mixing other pigments in addition to the pigment of the present invention, due to the characteristics of the pigment of the present invention of exhibiting color with large chroma even in a region with a small lightness, it is possible to reduce the kinds of the other pigments to be mixed, and, in that case, it is possible to make the cosmetic composition not easily allow color separation.

In addition, the iron oxide pigment of the present invention has a large coloring power and thus enables cosmetic compositions exhibiting a desired color at a smaller content than ever, which increases the degree of freedom in formulation. The blending of a tone modifying agent makes it possible for the iron oxide pigment of the present invention to adjust a subtle difference in the color even in dark color and to develop a wide range of colors. In addition, the cosmetic composition of the present invention can also be used as skin cosmetics oriented to colors that are not dark color. The use of the iron oxide pigment of the present invention makes it possible to provide a smooth texture or usability making application to wide ranges easy to the cosmetic composition.

Dark-color skin ordinarily refers to skin with a dark or deep shade. Impression imparted by human skin color is significantly affected by observers, environments, psychological effects, or the like; however, in the present specification, only for the purpose of defining the terminology to clarify the scope of one preferable aspect of the present invention, when a reflection of light is evaluated using a colorimeter, skin exhibiting a color having a value of lightness L of 30 or less in the Lab color space is defined as "dark-color skin." The above-described numerical range does not have any social implications. Whether or not an individual's skin matches the dark-color skin cannot be any basis for determining anything about the individual.

The cosmetic composition of the present invention can also be used as cosmetics other than skin cosmetics. The cosmetic composition of the present invention can be used as an alternative to brown cosmetic compositions that have been conventionally used.

(Form of Cosmetics)

A liquid cosmetic is one of the aspects of the cosmetic composition of the present invention. As the liquid form, the cosmetic composition may be in any state such as an aqueous dispersion, an oily dispersion, and a lotion, and can be made into, for example, makeup cosmetics such as makeup base, control color, sunscreen cosmetics, eye shadow, eyeliner, mascara, cheek color, and nail polish, skin care cosmetics, hair care cosmetics, permanent hair coloring agents, semi-permanent hair coloring agents, primary hair coloring agents, nail cosmetics, and the like.

A solid cosmetic is one of the aspects of the cosmetic composition of the present invention. As the solid form, the cosmetic composition may be in any state such as a powder, a solid powder, a paste, and an oily solid, and can be made into, for example, makeup cosmetics such as makeup base, foundation, loose powder, blush, concealer, eyebrow, face powder, control color, mascara, cheek color, body powder, pressed powder, perfume powder, and baby powder, skin care cosmetics, hair care cosmetics, and the like. In addition, the cosmetic composition can also be used as cosmetics for which it is not essential to add color, for example, facial cleansing powder, soap, powdered bath salt, and the like.

A gel cosmetic is one of the aspects of the cosmetic composition of the present invention. As the gel form, the cosmetic composition may be in any state such as a cream, an emulsion, an oily liquid, and a paste, and can be made into, for example, makeup cosmetics such as makeup base, control color, sunscreen cosmetics, lipstick, eye shadow, eyeliner, mascara, lip cream, lip color, lip gloss, and nail polish, skin care cosmetics, hair care cosmetics, and the like. In addition, the cosmetic composition can also be used as cosmetics for which it is not essential to add color, for example, shampoo, rinse, body shampoo, facial cleansing foam, peel-off pack, toothpaste, and the like.

EXAMPLES

Hereinafter, the present invention will be specifically described with examples, but the following examples will be simply shown for exemplification, and the scope of the invention is not limited by these by any means.

In mixing operations described in the examples and comparative examples, rotation speeds were appropriately adjusted so that all are uniformly mixed and droplets are not scattered around in consideration of properties relating to behaviors during mixing, such as the amount or viscosity of a mixture and the capacity of a device used. In addition, in a case where the same effect is obtained as long as a component is an ordinary commercially available product, such as sodium hexametaphosphate, regardless of a company that produces the product, the name of the company, which is the manufacturer or distributor, will be omitted.

[Example 1]

Yellow iron oxide (LL-100HP, manufactured by Titan Kogyo, Ltd.) synthesized by a known method was fired at 380°C in the air for one hour, the product was fired at 320°C in a hydrogen atmosphere for 4.5 hours, furthermore, fired at 250°C in the air for eight hours and cooled by an air, thereby obtaining an iron oxide pigment A.

[Example 2]

Black iron oxide ABL-209HP (manufactured by Titan Kogyo, Ltd.) synthesized by a known method was first fired at 80°C in the air for four hours, next, fired at 120°C for four hours, additionally, fired at 160°C for four hours, finally, fired at 200°C for four hours and cooled by an air, thereby obtaining an iron oxide pigment B.

[Example 3]

The same operation as in Example 2 was performed on black iron oxide BL-100HP (manufactured by Titan Kogyo, Ltd.) synthesized by a known method, thereby obtaining an iron oxide pigment C.

[Comparative Example 1]

500 g/kg of commercially available yellow iron oxide LL-100HP (manufactured by Titan Kogyo, Ltd., the element Fe content: 622 g/kg, the FeO content: 0 g/kg, and the $Fe_2O_3$ content: 887 g/kg) and 500 g/kg of red iron oxide R-516HP (manufactured by Titan Kogyo, Ltd., the element Fe content: 695 g/kg, the FeO content: 0 g/kg, and the $Fe_2O_3$ content: 994 g/kg) were uniformly mixed, thereby producing a mixed pigment 1. The content of the element Fe in the obtained mixed pigment 1 was 659 g/kg, the FeO content was 0 g/kg, and the $Fe_2O_3$ content was 941 g/kg.

[Comparative Example 2]

750 g/kg of commercially available yellow iron oxide LL-100HP, 150 g/kg of red iron oxide R-516HP, and 100 g/kg of black iron oxide BL-100HP (manufactured by Titan Kogyo, Ltd., the element Fe content: 707 g/kg, the FeO content: 220 g/kg, and the $Fe_2O_3$ content: 780 g/kg) were uniformly mixed, thereby producing a mixed pigment 2. The content of the element Fe in the obtained mixed pigment 2 was 641 g/kg, the FeO content was 22 g/kg, the $Fe_2O_3$ content was 892 g/kg, and the total content of FeO, $Fe_2O_3$ and FeO was 914 g/kg.

[Evaluation Items and Evaluation Methods]

Regarding the iron oxide pigments of Examples 1 to 3 and the mixed pigments of the comparative examples, the following items were evaluated.

[Content of Element Fe in Iron Oxide Pigment]

The content was evaluated by the method according to JIS K 5109. 15 mL of hydrochloric acid was added to 0.3 g of a well-dried iron oxide pigment, and the mixture was heated to be concentrated up to approximately 5 mL. A stannous chloride solution was silently added dropwise thereto while being heated, two droplets were further added thereto when the liquid turned colorless, and the liquid was rapidly cooled with flowing water. 10 mL of a mercuric chloride saturated solution was added thereto, the liquid was left to stand for five minutes, 20 mL of a mixed acid was added thereto, and pure water was added thereto to adjust the liquid amount to 50 mL. Five droplets of a diphenylamine-4-sulfonic acid sodium salt aqueous solution having a concentration of 2 g/kg was added thereto, the liquid was titrated with a 0.05 mol/L potassium dichromate solution, and when the color turned from green to violet was regarded as the end point. When the amount of the 0.05 mol/L potassium dichromate solution used is indicated by c (mL), the content (g/kg) of the element Fe in the pigment becomes 18.62 × c. The results are shown in Table 1.

[FeO Content and $Fe_2O_3$ Content]

The content was evaluated by the method according to JIS K 5109.

1) FeO Content

35 mL of distilled water and 15 mL of concentrated sulfuric acid were added to 0.4 g of a well-dried iron oxide pigment, heated to be dissolved, and cooled to room temperature with flowing water. 150 mL of pure water, 10 mL of phosphoric acid, five droplets of a diphenylamine-4-sulfonic acid sodium salt aqueous solution having a concentration of 2 g/kg were added thereto, the liquid was titrated with a 0.05 mol/L potassium dichromate solution, and when the color turned from light green to violet was regarded as the end point. When the amount of the 0.05 mol/L potassium dichromate solution used is indicated by a (mL), the FeO content (g/kg) in the pigment becomes 17.96 × a.

2) $Fe_2O_3$ Content

15 mL of hydrochloric acid was added to 0.4 g of a well-dried iron oxide pigment, and the mixture was heated to be concentrated up to approximately 5 mL. A stannous chloride solution was silently added dropwise thereto while being heated, two droplets were further added thereto when the liquid turned colorless, and the liquid was rapidly cooled with flowing water. 10 mL of a mercuric chloride saturated solution was added thereto, the liquid was left to stand for five minutes, 20 mL of a mixed acid was added thereto, and pure water was added thereto to adjust the liquid amount to 120 mL. Five droplets of a diphenylamine-4-sulfonic acid sodium salt aqueous solution having a concentration of 2 g/kg was added thereto, the liquid was titrated with a 0.05 mol/L potassium dichromate solution, and when the color turned from green to violet was regarded as the end point. When the amount of the 0.05 mol/L potassium dichromate solution used is indicated by b (mL), the $Fe_2O_3$ content (g/kg) becomes 19.96 × (b - a). The results are shown in Table 1.

Pb, As, Hg, and Cd were analyzed by ICP-MS after the iron oxide pigment was dissolved with sulfuric acid. Zn, Ba, Cr, Cu, and Ni were analyzed by ICP after the iron oxide pigment was dissolved with hydrochloric acid. The results are shown in Table 2.

[X-Ray Diffraction Intensity Ratio between Corundum Structure and Spinel Structure]

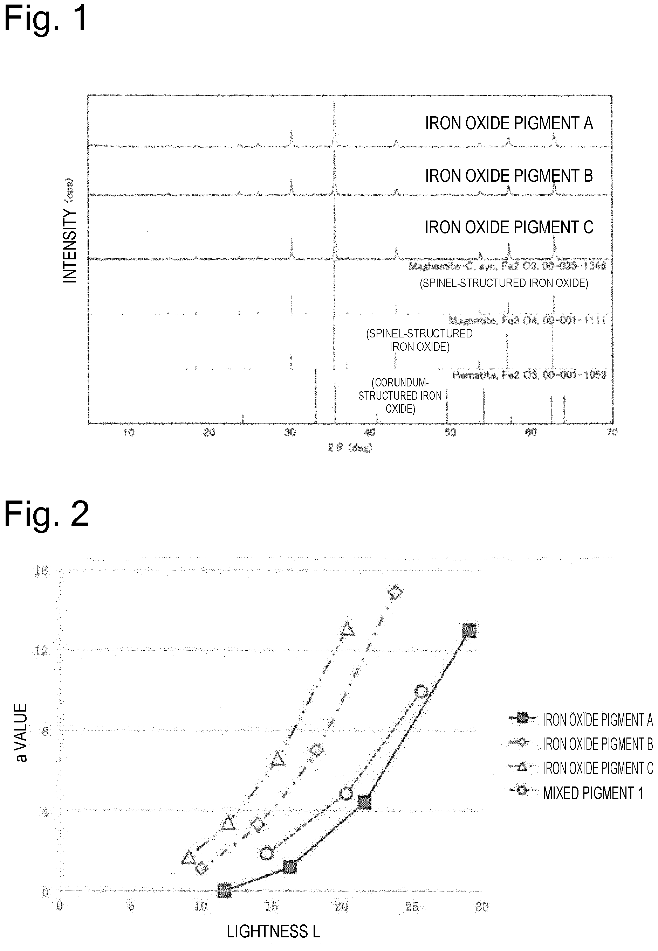

X-ray diffraction measurement was performed by a powder method using an X-ray diffractometer RINT-TTR III manufactured by Rigaku Corporation. The iron oxide pigment was ground with a mortar and packed in a cell in a quantity of approximately 0.7 g ± 0.2 g, the start angle was set to 5.0000°, the end angle was set to 70.0000°, the sampling width was set to 0.0100°, the scanning speed was set to 5.0000 °/min, the divergence slit was set to 0.5°, the scattering slit was set to 0.5°, the width of the light-receiving slit was set to 0.15 mm, for the characteristic X ray, copper was used as the negative electrode, and the wavelength was set to 0.15418 nm. On the obtained X-ray diffraction pattern, smoothing, background processing, and peak detection were performed using analysis software MDI JADE7 manufactured by Materials Data, Inc. The X-ray diffraction measurement results are shown in Fig. 1. The ratio of the integrated intensity of the diffraction lines of a corundum structure appearing in a range of 32.60° or more and 33.60° or less in a case where the integrated intensity of the diffraction lines of a spinel-type structure appearing in a range of 35.10° or more and 36.10° or less was regarded as 100.00 was calculated (in the present specification, this ratio will also be referred to as "the x-ray diffraction intensity ratio between the corundum structure and the spinel structure"). In a case where no peaks are detected in a range of 32.60° or more and 33.60° or less, the ratio is regarded as 0.00. The results are shown in Table 3.

[Average Short Axis Length of Primary Particles of Iron Oxide Pigment Particles]

The average short axis length was measured using a transmission electron microscope JEM-1400plus manufactured by JEOL Ltd. The observation magnification was set to 30,000 times (the observation magnification of the transmission electron microscope 10,000 times × print three times). The shortest axis lengths of approximately 300 primary particles in the observation visual field were evaluated using image analysis software ImageJ. The average value of the shortest axis lengths of approximately 300 particles was calculated and regarded as the average short axis length. The results are shown in Table 3.

[BET Specific Surface Area]

The BET specific surface area was measured by the single point BET method using GEMINI VII 2390 manufactured by Micromeritics Instrument Corporation. The results are shown in Table 3.

[Bulk Density]

The bulk density was evaluated by the method according to JIS K 5101-12-1. A funnel and a sieve with a mesh size of 45 $\mu$m were placed on a funnel stand including a powder tester manufactured by Hosokawa Micron Corporation, one tablespoon (capacity: 15 mL) of theiron oxide pigment was placed on the sieve, the entire surface of the sieve was lightly swept evenly with a hard bristle brush to disperse and drop the pigment, and the pigment was received in a receiver below the funnel stand. This operation was repeated until the pigment was piled up in the receiver. Next, the mountain portion was scraped off with a spatula, and then the mass of the contents of the receiver was measured. When the mass of the contents is indicated by d (g), the bulk density (g/mL) becomes d/30. The results are shown in Table 3.

[Residue on Sieve]

The residue on sieve was evaluated by the method according to JIS K 5101-14-1. 1.00 g of the iron oxide pigment was dispersed in a 0.5 g/kg sodium hexametaphosphate aqueous solution and then passed through a sieve with a mesh size of 45 $\mu$m. The sieve was washed with a small amount of flowing water, furthermore, the sieve was washed with a small amount of ethanol and dried at 105°C for one hour, and the mass of the pigment remaining on the sieve was measured to calculate the ratio of the pigment remaining on the sieve. The results are shown in Table 3.

[Oil Absorption]

2 g of the iron oxide pigment was spread on a glass plate, boiled linseed oil was added dropwise to the center of the pigment little by little, and, each time, the entire was mixed with a spatula to knead the pigment and the boiled linseed oil. A point in time where the entire turned into uniform paste-like clumps was regarded as the end point. When the boiled linseed oil used is indicated by e (mL), the oil absorption (g/100 g) becomes 46.75 × e. The results are shown in Table 3.

[a Value, b Value, and Chroma]

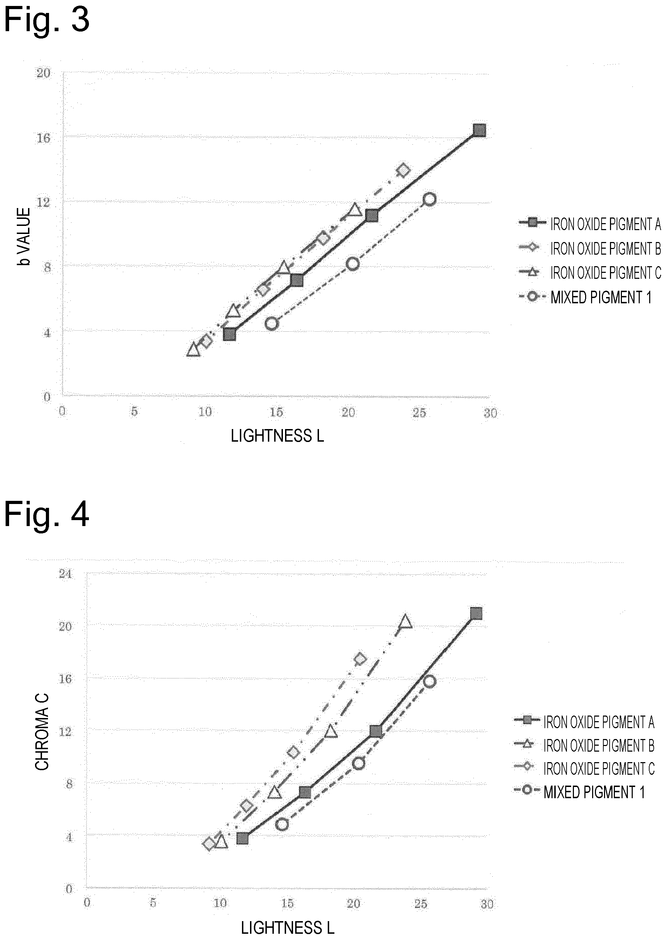

For the iron oxide pigments A to C and the mixed pigment 1, a powder having a reduced lightness was prepared by substituting 250 g/kg of each pigment with black iron oxide BL-100HP. 834 g/kg of ALUKIDIR® 4250 manufactured by DIC Corporation, 156 g/kg of xylene, and 10 g/kg of butan-2-one=oxime were mixed together, and this was regarded as a solvent 1. 0.5 g of each powder was placed on the lower plate of a Hoover muller manufactured by Toyo Seiki Seisaku-sho, Ltd., 1 mL of the solvent 1 was added dropwise thereto, the powder and the solvent 1 were kneaded under the application of a load of 667 N until the entire became a uniform paste form, furthermore, 2 mL of the solvent 1 was added thereto, and the components were mixed. An obtained slurry was applied to test paper, left still for 30 minutes, and then baked at 130°C in the air for 30 minutes, and the L value, a value, and b value of the baked coated film were measured using an SM-7 color tester manufactured by Suga Test Instruments Co., Ltd. (hereinafter, referred to as "color tester"). For a pigment obtained by substituting 500 g/kg or 750 g/kg of each pigment with black iron oxide, the L value, the a value, and the b value were measured in the same manner. The obtained results are shown in Fig. 2 and Fig. 3. When the obtained a value and b value are indicated by $a_1$ and $b_1$, respectively, the chroma C is calculated by the following equation:

$$C = (a_1^2 + b_1^2)^{1/2}$$

The obtained results are shown in Fig. 4.

[Coloring Power]

For the iron oxide pigments A to C and the mixed pigment 2, a mixed powder was prepared by mixing 0.125 g of each pigment and 0.375 g of titanium oxide. Incidentally, 834 g/kg of ALUKIDIR® 4250 manufactured by DIC Corporation, 156 g/kg of xylene, and 10 g/kg of butan-2-one=oxime were mixed together, and this was regarded as a solvent 1. 0.5 g of each powder was placed on the lower plate of a Hoover muller manufactured by Toyo Seiki Seisaku-sho, Ltd., 1 mL of the solvent 1 was added dropwise thereto, the powder and the solvent 1 were kneaded under the application of a load of 667 N until the entire became a uniform paste form, furthermore, 2 mL of the solvent 1 was added thereto, and the components were mixed. An obtained slurry was applied to test paper, left still for 30 minutes, and then baked at 130°C in the air for 30 minutes, and the L value, a value, and b value of the baked coated film were measured using a color tester. A color difference ΔE between the measured values of the L value, the a value, and the b value and white (L = 100, a = 0, and b = 0) was regarded as the coloring power. When the measured values of the L value, the a value, and the b value are indicated by $L_2$, $a_2$, and $b_2$, respectively, the coloring power ΔE is calculated by the following equation:

$$\Delta E = \{(L_2 - 100)^2 + (a_2 - 0)^2 + (b_2 - 0)^2\}^{1/2}$$

The results are shown in Table 4.

[Table 1]

|  | Element Fe content | FeO content | Fe$_2$O$_3$ content | Total content of Fe$_2$O$_3$ and FeO |
|---|---|---|---|---|
|  | g /kg | g /kg | g /kg | g /kg |
| Iron oxide pigment A | 693 | 0 | 991 | 991 |
| Iron oxide pigment B | 685 | 0 | 980 | 980 |
| Iron oxide pigment C | 688 | 4 | 979 | 983 |
| Mixed pigment 1 | 659 | 0 | 941 | 941 |
| Mixed pigment 2 | 641 | 22 | 892 | 914 |

[Table 2]

|  | Pb content | As content | Hg content | Cd content | Zn content | Ba content | Cr content | Cu content | Ni content |
|---|---|---|---|---|---|---|---|---|---|
|  | mg /kg | mg /kg | m g /kg | mg /kg | mg /kg | mg /kg | mg /kg | mg /kg | mg /kg |
| Iron oxide pigment A | 2 | 2 | 1 | 1 | 20 | 5 | 20 | 10 | 5 |
| Iron oxide pigment B | 2 | 2 | 1 | 1 | 20 | 5 | 20 | 10 | 5 |
| Iron oxide pigment C | 2 | 2 | 1 | 1 | 20 | 5 | 20 | 10 | 5 |
| Mixed pigment 1 | 2 | 2 | 1 | 1 | 2 0 | 5 | 20 | 10 | 5 |

(continued)

|  | Pb content | As content | Hg content | Cd content | Zn content | Ba content | Cr content | Cu content | Ni content |
|---|---|---|---|---|---|---|---|---|---|
|  | mg /kg | mg /kg | m g /kg | mg /kg | mg /kg | mg /kg | mg /kg | mg /kg | mg /kg |
| Mixed pigment 2 | 2 | 2 | 1 | 1 | 20 | 5 | 20 | 10 | 5 |

[Table 3]

|  | X-ray diffraction intensity ratio between corundum structure and spinel structure | Average short axis length | BET specific surface area | Bulk density | Residue on sieve | Oil absorption |
|---|---|---|---|---|---|---|
|  | - | nm | $m^2$ /g | g /mL | g /kg | g /100g |
| Iron oxide pigment A | 0.00 | 110 | 12.8 | 39 | 0.19 | 36 |
| Iron oxide pigment B | 3.54 | 156 | 8.4 | 28 | 0.13 | 40 |
| Iron oxide pigment C | 0.00 | 280 | 5.4 | 38 | 0.06 | 32 |

[Table 4]

|  | Coloring power ΔE | |
|---|---|---|
| Iron oxide pigment A | 55. | 50 |
| Iron oxide pigment B | 55. | 99 |
| Iron oxide pigment C | 53. | 03 |
| Mixed pigment 2 | 51. | 66 |

It is found from Fig. 1 that the iron oxide pigments of Examples 1 to 3 have a spinel structure or have a spinel structure and a corundum structure. In addition, it is found from Fig. 2 and Fig. 3 that the iron oxide pigment A of Example 1 has a larger b value compared with the mixed pigment 1 of Comparative Example 1 at the same lightness (L value). Such an iron oxide pigment has an advantage of easily toning yellow-based color particularly in a region with a small lightness. In addition, it is found that the iron oxide pigments B and C of Examples 2 and 3 have an a value and a b value that are both larger than those of the mixed pigment 1. Such an iron oxide pigment has an advantage of easily toning a wide range of color in a region with a small lightness. Furthermore, it is found from Fig. 4 that the iron oxide pigments used in all of the examples exhibit larger chroma compared with the mixed pigment 1 at the same lightness (L value).

From the above-described results, it is found that the iron oxide pigment of the present invention has a large a value and/or a large b value and large chroma in a region with a small lightness and has a large coloring power. Cosmetic compositions into which the iron oxide pigment of the present invention has been blended can be said to be suitable for use as skin cosmetics oriented to dark color.

A skin cosmetic is one of the main aspects of the present invention. Hereinafter, formulation examples will be described.

[Formulation Examples]

| Formulation 1 | Light Beige Powder Foundation |
|---|---|
| Component | blending proportions (g/kg) |
| (1) Iron oxide pigment A | 9.4 |
| (2) Black iron oxide pigment (BL-100HP manufactured by Titan Kogyo, Ltd.; also in the following formulation examples, BL-100HP was used as black iron oxide) | 0.6 |
| (3) Mica (PDM-1000 manufactured by Topy Industries Limited) | 490 |
| (4) Talc (JA-46R manufactured by Asada Milling Co., Ltd.) | 300 |
| (5) Titanium oxide (C-50R manufactured by Ishihara Sangyo Kaisha, Ltd.) | 100 |
| (6) Oil {obtained by mixing CRODALAN SWL (manufactured by Croda International Plc), PHYTOSQUALAN (manufactured by Iwase Cosfa Co., Ltd.), O. D. O (manufactured by The Nisshin OilliO Group, Ltd.), T. I. O (manufactured by The Nisshin OilliO Group, Ltd.), and KF-56 (manufactured by Shin-Etsu Chemical Co., Ltd.) in proportions of 4:4:3:3:6} | 100 |

(Manufacturing Method)

(1) and (2) were manually mixed in advance, and a mixed pigment and (3) to (6) were mixed uniformly with FM-10B manufactured by Mitsui Miike Machinery Company, Limited (hereinafter, referred to as "Henschel mixer"). A mixture was pulverized with a hammer mill, loaded into a mold, and compression-molded, thereby obtaining an intended light beige powder foundation. The obtained light beige powder foundation had a smooth texture. In addition, importantly, the light beige powder foundation had a natural look while imparting a healthy appearance in a vivid color.

| Formulation 2 | Beige Brown Powder Foundation |
|---|---|
| Component | blending proportions (g/kg) |
| (1) Iron oxide pigment B | 248 |
| (2) Black iron oxide | 16 |
| (3) Mica (PDM-1000 manufactured by Topy Industries Limited) | 264 |
| (4) Sericite | 352 |
| (5) Dimethicone (Manufacturing Method) | 120 |

(1) and (2) were manually mixed in advance, and a mixed pigment and (3) to (5) were mixed more uniformly with a Henschel mixer. A mixture was pulverized with a hammer mill, loaded into a mold, and compression-molded, thereby obtaining an intended beige brown powder foundation. The obtained beige brown powder foundation had a smooth texture. In addition, importantly, the beige brown powder foundation had a natural look while imparting a healthy appearance in a vivid color.

| Formulation 3 Dark | Brown Powder Foundation |
|---|---|
| Component | blending proportions (g/kg) |
| (1) Iron oxide pigment C | 300 |
| (2) Black iron oxide | 300 |
| (3) Mica (PDM-1000 manufactured by Topy Industries Limited) | 300 |

(continued)

| Formulation 3 Dark | Brown Powder Foundation |
|---|---|
| (4) Oil {obtained by mixing CRODALAN SWL (manufactured by Croda International Plc), PHYTOSQUALAN (manufactured by Iwase Cosfa Co., Ltd.), O. D. O (manufactured by The Nisshin OilliO Group, Ltd.), T. I. O (manufactured by The Nisshin OilliO Group, Ltd.), and KF-56 (manufactured by Shin-Etsu Chemical Co., Ltd.) in proportions of 4:4:3:3:6} | 100 |

(Manufacturing Method)

(1) and (2) were manually mixed in advance, and a mixed pigment, (3), and (4) were mixed more uniformly with a Henschel mixer. A mixture was pulverized with a hammer mill, loaded into a mold, and compression-molded, thereby obtaining an intended dark brown powder foundation. The obtained dark brown powder foundation had a smooth texture. In addition, importantly, the dark brown powder foundation had a natural look while imparting a healthy appearance in a vivid color.

| Formulation 4 | Stick-Form Foundation |
|---|---|
| Component | blending proportions (g/kg) |
| (1) Cyclopentasiloxane | 300 |
| (2) Ethylhexyl methoxycinnamate | 50 |
| (3) Diisostearyl malate | 40 |
| (4) Candelilla wax | 60 |
| (5) Hydrogenated jojoba ester | 40 |
| (6) Cetyl dimethicone copolyol | 20 |
| (7) Sorbitan sesquiisostearate | 5 |
| (8) Hydrogen dimethicone-treated iron oxide pigment A | 25 |
| (9) Hydrogen dimethicone-treated titanium oxide | 83 |
| (10) Hydrogen dimethicone-treated talc | 60 |
| (11) Methyl methacrylate crosspolymer (M-305 manufactured by Matsumoto Yushi-Seiyaku Co., Ltd.) | 40 |
| (12) Purified water | Residue |
| (13) Sodium citrate | 3 |
| (14) Propanediol | 30 |
| (15) Glycerin | 20 |

(Manufacturing Method)

Powder portions (8) to (11) were mixed in advance with a Henschel mixer. Oil-phase portions (1) to (7) were weighed in a container where the total amount was to be prepared, and heated and dissolved. Water-phase portions (12) to (15) were weighed in a separate container, heated, and dissolved. The powder portions were added to and uniformly dispersed in the oil-phase portions, the water-phase portions were added thereto, emulsified, and defoamed, then, the bulk was made to flow into a mold and slowly cooled to room temperature, thereby obtaining an intended stick-form foundation. The obtained stick-form foundation had excellent adhesion and a smooth feeling of use. In addition, importantly, the stick-form foundation had a natural look while imparting a healthy appearance in a vivid color to users.

| Formulation 5 | W/O Emulsifying Foundation | |
|---|---|---|
| Component | | blending proportions (g/kg) |
| (1) PEG-10 dimethicone | | 20 |
| (2) Polyglyceryl-10 polyricinoleate | | 5 |
| (3) Neopentyl glycol dicaprate | 30 | |
| (4) Squalane | 10 | |
| (5) Pentaerythrityl tetraoctanoate | 20 | |
| (6) Stearoyl inulin | 10 | |

(continued)

| Formulation 5 | W/O Emulsifying Foundation |
| --- | --- |
| Component | blending proportions (g/kg) |
| (7) Ethylhexyl methoxycinnamate | 40 |
| (8) Cyclopentasiloxane | Residue |
| (9) Hydrogen dimethicone-treated titanium oxide | 77 |
| (10) Hydrogen dimethicone-treated talc | 57 |
| (11) Hydrogen dimethicone-treated iron oxide pigment C | 27 |
| (12) Purified water | 380 |
| (13) 1,3-Butylene glycol | 60 |
| (14) Glycerin | 10 |
| (15) Sodium chloride | 10 |
| (16) Phenoxyethanol | 5 |

(Manufacturing Method)

Powder portions (9) to (11) stirred and mixed in advance with a Henschel mixer were added to oil-phase portions (1) to (8) and uniformly dispersed with a stirrer. Water-phase portions (12) to (16) were heated and dissolved in a separate container. The water-phase portions were added to the oil-phase portions in which the powder portions had been dispersed, emulsified, and then cooled to room temperature, thereby obtaining an intended W/O emulsifying foundation. The obtained W/O emulsifying foundation was easy to spread and had a smooth feeling of use. In addition, importantly, the W/O emulsifying foundation had a natural look while imparting a healthy appearance in a vivid color to users.

| Formulation 6 | O/W Emulsifying Foundation | |
| --- | --- | --- |
| Component | | blending proportions (g/kg) |
| (1) Stearic acid | | 10 |
| (2) Isostearic acid | | 3 |
| (3) Cetyl ethylhexanoate | | 40 |
| (4) Liquid paraffin 70cs | | 110 |
| (5) Steareth-10 | | 20 |
| (6) Cetyl alcohol | | 15 |
| (7) Triethoxycaprylylsilane-treated talc | | 50 |
| (8) Triethoxycaprylylsilane-treated iron oxide pigment A | | 27 |
| (9) Triethoxycaprylylsilane-treated titanium oxide | | 75 |
| (10) Triethanolamine | | 12 |
| (11) Propanediol | | 50 |
| (12) Xanthan gum | | 2 |
| (13) Purified water | | Residue |
| (14) Phenoxyethanol | | 5 |

(Manufacturing Method)

Mixed and pulverized (7) to (9) were added to and uniformly dispersed in (1) to (6) that had been heated and dissolved at 85°C. A mixture of (10) to (14) heated to 85°C was slowly added thereto to perform emulsification, an emulsified product was stirred, cooled to room temperature, and then loaded into an appropriate container, thereby obtaining an intended O/W emulsifying foundation. The obtained O/W emulsifying foundation was easy to spread and had a smooth feeling of use. In addition, importantly, the O/W emulsifying foundation had a natural look while imparting a healthy appearance in a vivid color to users.

| Formulation 7 | Two-Way Cake Foundation | |
|---|---|---|
| Component | | blending proportions (g/kg) |
| (1) Dimethicone-treated talc | | Residue |
| (2) Dimethicone-treated titanium oxide | | 100 |
| (3) Dimethicone-treated mica | | 200 |
| (4) Dimethicone-treated sericite | | 360 |
| (5) Nylon powder | | 100 |
| (6) Dimethicone-treated red iron oxide (R-516HP manufactured by Titan Kogyo, Ltd.; even in the following formulations, R-516HP was used as red iron oxide) | | 2 |
| (7) Dimethicone-treated iron oxide pigment B | 27 | |
| (8) Dimethicone 1000cs | 60 | |
| (9) Isotridecyl isononanoate | 30 | |
| (10) Squalane | 30 | |
| (11) Tocopherol | 1 | |
| (12) 1,3-Butylene glycol | 10 | |

(Manufacturing Method)

(1) to (7) were mixed together with a Henschel mixer, (8) to (12) heated and dissolved were mixed therewith, then, a mixture was pulverized with an atomizer and press-molded in an aluminum dish, thereby obtaining an intended two-way foundation. The obtained two-way foundation was smooth and had a non-sticky feeling of use. In addition, importantly, the two-way foundation had a natural look while imparting a healthy appearance in a vivid color to users.

| Formulation 8 | Oily Cake Foundation |
|---|---|
| Component | blending proportions (g/kg) |
| (1) Dimethicone-treated talc | Residue |
| (2) Dimethicone-treated titanium oxide | 130 |
| (3) Dimethicone-treated sericite | 280 |
| (4) Dimethicone-treated iron oxide pigment A | 27 |
| (5) Dimethicone-treated black iron oxide | 1 |
| (6) Candelilla wax | 10 |
| (7) Carnauba wax | 10 |
| (8) Ceresin | 15 |
| (9) Cyclopentasiloxane | 140 |
| (10) Isononyl isononanoate | 250 |
| (11) Polyglyceryl diisostearate | 20 |
| (12) Ethylhexyl methoxycinnamate | 30 |

(Manufacturing Method)

Powder portions (1) to (5) were mixed with a Henschel mixer and uniformly pulverized. Oil-phase portions (6) to (12) were heated and dissolved, and the powder portions were added thereto and uniformly stirred. After defoaming, the bulk was made to flow into a tray and slowly cooled to room temperature, thereby obtaining an intended oily cake foundation. The obtained oily cake foundation had no oiliness and a smooth feeling of use. In addition, importantly, the oily cake foundation had a natural look while imparting a healthy appearance in a vivid color to users.

| Formulation 9 | Blush |
|---|---|
| Component | blending proportions (g/kg) |
| (1) Triethoxycaprylylsilane-treated talc | 250 |
| (2) Triethoxycaprylylsilane-treated sericite | Residue |
| (3) Stearic acid-treated fine particle titanium oxide (STV-455 manufactured by TitanKogyo, Ltd.) | 30 |
| (4) Triethoxycaprylylsilane-treated titanium oxide | 18 |
| (5) Triethoxycaprylylsilane-treated black iron oxide | 0.5 |
| (6) Triethoxycaprylylsilane-treated red iron oxide | 2 |
| (7) Triethoxycaprylylsilane-treated iron oxide pigment B | 8 |
| (8) Ethylhexyl methoxycinnamate | 30 |
| (9) Ethylhexyl palmitate | 50 |
| (10) Preservative | Appropriate amount |
| (11) Antioxidant | Appropriate amount |

(Manufacturing Method)

(1) to (7) were mixed together with a Henschel mixer, (8) to (11) heated and dissolved were mixed therewith, and then a mixture was pulverized with an atomizer. This was press-molded in an aluminum dish, thereby obtaining an intended blush. The obtained blush had excellent usability. In addition, importantly, the blush had a natural look while imparting a healthy appearance in a vivid color to users.

| Formulation 10 Component | Loose Powder blending proportions (g/kg) |
|---|---|
| (1) Talc | Residue |
| (2) Pigment-grade titanium | oxide 10 |
| (3) AMIHOPE® LL | 30 |
| (4) PMMA (Matsumoto Microsphere S-100 manufactured by MatsumotoYushi-Seiyaku Co., Ltd., 10 $\mu$m product) | 80 |
| (5) Preservative | Appropriate amount |
| (6) Iron oxide pigment B | 10 |
| (7) Squalane | 10 |
| (8) Preservative | Appropriate amount |
| (9) Antioxidant | Appropriate amount |
| (10) Perfume | Appropriate amount |

(Manufacturing Method)

(1) to (7) were mixed and pulverized, this mixed and pulverized product was transferred to LAB. MIXER LM-110T manufactured by Hanil Electric. Co., Ltd. (8) to (10) were added thereto, and the components were stirred and mixed to become uniform. The obtained mixture was pulverized with a sample mill TASM-1 manufactured by Tokyo Atomizer M.F.G. Co., Ltd., and this was loaded to obtain a loose powder. The obtained loose powder had a natural look while imparting a healthy appearance in a vivid color to users.

A cosmetic having a liquid form is also one of the aspects of the present invention. Hereinafter, formulation examples will be described.

| Formulation 11 | Aqueous Eyeliner |
|---|---|
| Component | blending proportions (g/kg) |
| (1) Black iron oxide | 50 |
| (2) Iron oxide pigment C | 25 |
| (3) Purified water | Residue |
| (4) Decaglyceryl laurate | 10 |
| (5) Glycerin | 60 |

(continued)

| Formulation 11 Component | Aqueous Eyeliner blending proportions (g/kg) |
|---|---|
| (6) Aqueous solution of 100 g/kg of carboxymethyl cellulose | 180 |
| (7) Phenoxyethanol | 5 |
| (8) Pentylene glycol | 10 |
| (9) Vinyl acetate resin emulsion (VINYBLAN® GV-5651 manufactured by NissinChemical Co., Ltd.) | 450 |

(Manufacturing Method)

(1) to (3) were weighed, and (1) and (2) were finely dispersed in (3) with a bead mill. (4) to (8) were weighed in a separate container, a pigment portion was added thereto and uniformly dispersed at 70°C, the components were cooled to room temperature, and (9) was added thereto, thereby obtaining an intended aqueous eyeliner. The obtained aqueous eyeliner exhibited a vivid brown color and had an excellent feeling of use.

| Formulation 12 Component | Nail Enamel blending proportions (g/kg) |
|---|---|
| (1) Nitrocellulose (viscosity: 1/2 seconds) | 100 |
| (2) Modified alkyd resin | 100 |
| (3) Acetyl tributyl citrate | 50 |
| (4) Ethyl acetate | 200 |
| (5) Ethanol | 50 |
| (6) Toluene | Residue |
| (7) Iron oxide pigment A | 90 |
| (8) Organic modified montmorillonite | 30 |

(Manufacturing Method)

(7) was mixed with a part of (2) and (3) and well kneaded. The residue of (2) and (3), and (1), (4) to (6), and (8) were added thereto and mixed until becoming uniform, thereby obtaining an intended nail enamel. The obtained nail enamel exhibited a vivid color and also had an excellent masking property.

| Formulation Example 13 Component | Primary Hair Coloring Agent blending proportions (g/kg) |
|---|---|
| (1) Iron oxide particle A | 50 |
| (2) Red No. 202 | 10 |
| (3) Polyvinyl alcohol 500 | 5 |
| (4) Triethanolamine | 6 |
| (5) Ethyl acetate | Residue |

(Manufacturing Method)

(3) to (5) were stirred with a Henschel mixer, (1) and (2) were added thereto when the components became uniform, and the components were stirred until all became uniform, thereby obtaining an intended primary hair coloring agent. The obtained primary hair coloring agent exhibited a brown vivid color and had no coarse texture.

A cosmetic having a solid form is also one of the aspects of the present invention. Hereinafter, formulation examples will be described.

| Formulation Example 14 Component | Press Type Eye Shadow blending proportions (g/kg) |
|---|---|
| (1) Iron oxide pigment C | 270 |
| (2) Sericite | Residue |
| (3) Talc | 149 |

(continued)

| Formulation Example 14 | Press Type Eye Shadow |
|---|---|
| Component | blending proportions (g/kg) |
| (4) Titanium mica (manufactured by BASF: Flamenco Super Pearl 120C) | 200 |
| (5) Methylparaben | 1 |
| (6) Dimethicone 1000cs | 70 |
| (7) Diisostearyl malate | 30 |
| (8) Isopropyl alcohol | Appropriate amount |

(Manufacturing Method)

(1) to (5) were mixed and pulverized. Next, (6) and (7) mixed together were added to a separate container and stirred and mixed to become uniform. An adjusted mixture was dispersed in (8) so as to be appropriately viscous, and this was compressed and loaded into an appropriate gold dish with a slurry loading machine. The mixture was dried at 40°C for 12 hours, thereby obtaining an intended press type eye shadow. The obtained product exhibited a vivid color and had excellent adhesion and a smooth feeling of use.

| Formulation 15 | Eyebrow Pencil |
|---|---|
| Component | blending proportions (g/kg) |
| (1) Black iron oxide | 160 |
| (2) Iron oxide pigment | C 25 |
| (3) Titanium oxide | 45 |
| (4) Talc | Residue |
| (5) Kaolin | 150 |
| (6) Japan wax | 200 |
| (7) Stearic acid | 100 |
| (8) Beeswax | 50 |
| (9) Hydrogenated castor oil | 50 |
| (10) Vaseline | 30 |
| (11) Lanolin | 30 |
| (12) Squalane | 30 |
| (13) Preservative | Appropriate amount |
| (14) Antioxidant | Appropriate amount |

(Manufacturing Method)

(1) to (5) mixed and pulverized were added to (6) to (14), and heated and dissolved, uniformly kneaded with a triple roll mill, and molded into a core, and made into a pencil shape by inserting the core into wood, thereby obtaining an intended eyebrow pencil. The obtained eyebrow pencil exhibited a vivid color and had a smooth feeling of use.

| Formulation 16 | Pressed Eyebrow |
|---|---|
| Component | blending proportions (g/kg) |
| (1) Titanium oxide | 140 |
| (2) Black iron oxide | 140 |
| (3) Iron oxide pigment C | 25 |
| (4) Ultramarine | 5 |
| (5) Mica | Residue |
| (6) Talc | 100 |
| (7) Lanolin wax | 100 |
| (8) Liquid paraffin | 40 |
| (9) Glyceryl stearate | 10 |
| (10) Preservative | Appropriate amount |
| (11) Antioxidant | Appropriate amount |

(Manufacturing Method)

(1) to (6) mixed and pulverized were added to (7) to (11) that had been heated and dissolved, mixed until becoming uniform, and compression-molded, thereby obtaining an intended pressed eyebrow. The obtained pressed eyebrow exhibited a vivid color and also had an excellent coloring power.

| Formulation 17 | Pressed Powder |
| --- | --- |
| Component | blending proportions (g/kg) |
| (1) Hydrogen dimethicone-treated | talc Residue |
| (2) Methyl methacrylate crosspolymer (M-305 manufactured by MatsumotoYushi-Seiyaku Co., Ltd.) | 100 |
| (3) Hydrogen dimethicone-treated sericite | 500 |
| (4) Silica (SUNSPHERE (R) NP-30 manufactured by AGC Si-Tech Co., Ltd.) | 60 |
| (5) Hydrogen dimethicone-treated titanium oxide | 55 |
| (6) Hydrogen dimethicone-treated black iron oxide | 0.5 |
| (7) Hydrogen dimethicone-treated red iron oxide | 0.5 |
| (8) Hydrogen dimethicone-treated iron oxide pigment B | 10 |
| (9) Ethylhexyl methoxycinnamate | 30 |
| (10) Squalane | 20 |
| (11) Preservative | Appropriate amount |
| (12) Antioxidant | Appropriate amount |

(Manufacturing Method)

Powder portions (1) to (8) were mixed and pulverized, these were transferred to a Henschel mixer, oil-phase portions (9) to (12) were added thereto, and the components were stirred and mixed to become uniform. After that, a mixture was pulverized with an atomizer. This was press-molded in an aluminum dish, thereby obtaining an intended pressed powder. The obtained pressed powder exhibited a vivid color and had excellent adhesion and, furthermore, a soft feeling of use.

A cosmetic having a gel form is also one of the aspects of the present invention. Hereinafter, formulation examples will be described.

| Formulation 18 | Cream Eye Shadow |
| --- | --- |
| Component | blending proportions (g/kg) |
| (1) Talc | 100 |
| (2) Kaolin | 50 |
| (3) Iron oxide pigment C | 45 |
| (4) Stearic acid | 30 |
| (5) Isopropyl myristate | 80 |
| (6) Liquid paraffin 70cs | 50 |
| (7) Propylene glycol laurate | 30 |
| (8) Tocopherol | 0.5 |
| (9) Purified water | Residue |
| (10) Concentrated glycerin | 50 |
| (11) 1,3-Butylene glycol | 10 |
| (12) Methylparaben | 2 |
| (13) Triethanolamine | 12 |
| (14) EDTA-3Na | 0.5 |

(Manufacturing Method)

(1) to (3) mixed and pulverized were added to (9) to (15), and stirred and mixed. (4) to (8) warmed and dissolved at 70°C to 80°C were added to this, emulsified, stirred, and cooled, thereby obtaining an intended cream eye shadow. The obtained cream eye shadow exhibited a vivid color and had smooth usability and also excellent adhesion.

| Formulation 19 | Oily Eyeliner |
|---|---|
| Component | blending proportions (g/kg) |
| (1) Dextrin isostearate (UNIFILMA® HVY manufactured by Chiba Flour Milling Co., Ltd.) | 50 |
| (2) Microcrystalline wax | 50 |
| (3) Dextrin palmitate | 100 |
| (4) Diisostearyl malate | 100 |
| (5) Isododecane | Residue |
| (6) Dimethicone-treated black iron oxide | 245 |
| (7) Dimethicone-treated iron oxide pigment B | 25 |
| (8) Mica | 200 |

(Manufacturing Method)

(1) to (5) were weighed, heated and mixed until becoming uniform. Furthermore, (6) to (8) mixed and pulverized were added thereto, heated and mixed until becoming uniform, cooled to 40°C, loaded into an appropriate container, and cooled to room temperature, thereby obtaining an intended oily eyeliner. The obtained oily eyeliner exhibited a vivid color and also had excellent elongation.

| Formulation 20 | Emulsifying Mascara |
|---|---|
| Component | blending proportions (g/kg) |
| (1) Purified water | Residue |
| (2) Polyvinylpyrrolidone | 20 |
| (3) Propanediol | 20 |
| (4) Aqueous solution of 1% cationized cellulose | 100 |
| (5) Bentonite | 5 |
| (6) Triethanolamine | 17 |
| (7) Methylparaben | 2 |
| (8) Talc | 40 |
| (9) Black iron oxide | 90 |
| (10) Iron oxide pigment C | 25 |
| (11) Carnauba wax | 55 |
| (12) Beeswax | 90 |
| (13) Stearic acid | 20 |
| (14) Self-emulsifying form of glyceryl stearate | 20 |
| (15) Propylene glycol stearate | 20 |
| (16) Hydrogenated polyisobutene | 20 |
| (17) Cyclopentasiloxane | 40 |
| (18) Acrylic resin emulsion (DAITOSOL® 5000AD manufactured by Daito Kasei Kogyo Co., Ltd.) | 200 |

(Manufacturing Method)

(8) to (10) stirred and mixed in advance with a Henschel mixer were added to (1) to (7) and uniformly dispersed with a stirrer. (11) to (17) heated and dissolved were added to this, emulsified, and then cooled to 40°C, (18) was added thereto, and the components were cooled to room temperature, thereby obtaining an intended emulsifying mascara. The obtained emulsifying mascara exhibited a vivid color and had an excellent feeling of use.

| Formulation 21 | Oily Mascara |
|---|---|
| Component | blending proportions (g/kg) |
| (1) Hydrogenated polyisobutene | 20 |

(continued)

| Formulation 21 | Oily Mascara |
|---|---|
| Component | blending proportions (g/kg) |
| (2) Isododecane | Residue |
| (3) Dextrin isostearate | 50 |
| (4) Dextrin palmitate | 150 |
| (5) Microcrystalline wax | 20 |
| (6) Polyethylene wax | 30 |
| (7) Propylparaben | 1 |
| (8) Talc | 60 |
| (9) Black iron oxide | 55 |
| (10) Iron oxide pigment B | 10 |
| (11) Silica dimethyl silylate | 5 |
| (12) Nylon fiber (5D-8mm manufactured by cosmaterials) | 10 |

(Manufacturing Method)

(8) to (10) mixed and pulverized in advance and (11) were mixed with (1) to (7) heated and dissolved. After cooling, (12) was uniformly dispersed, and an oily mascara was obtained. The obtained oily mascara exhibited a vivid color and also had excellent adhesion and usability.

| Formulation 22 | Lipstick |
|---|---|
| Component | blending proportions (g/kg) |
| (1) Ceresin | 100 |
| (2) Microcrystalline wax | 32 |
| (3) Paraffin | 50 |
| (4) Pentaerythrityl tetraisostearate | 200 |
| (5) Diisostearyl malate | 150 |
| (6) Hydrogenated polydecene | 100 |
| (7) Vaseline | 100 |
| (8) Polyglyceryl-2 triisostearate | Residue |
| (9) Simethicone | 1 |
| (10) Propylparaben | 1 |
| (11) Red No. 202 | 4 |
| (12) Iron oxide pigment A | 18 |
| (13) Titanium oxide | 3 |
| (14) Silica | 10 |

(Manufacturing Method)

(1) to (14) were weighed, and heated and mixed. The components were uniformly dispersed with a triple roll, then, further heated, and uniformly stirred. After defoaming, a heated mixture was made to flow into a die and rapidly cooled. The mixture was mounted in an appropriate container, thereby obtaining an intended lipstick. The obtained lipstick exhibited a bright red unique color.

| Formulation 23 | Lip Color |
|---|---|
| Component | blending proportions (g/kg) |
| (1) Dextrin palmitate/ethylhexanoate | 50 |
| (2) Pentaerythrityl tetraisostearate | 120 |
| (3) Hydrogenated polydecene | 100 |
| (4) Polyglyceryl-2 triisostearate | Residue |
| (5) Pentaerythrityl hydrogenated rosinate | 100 |
| (6) Octyldodecyl isostearate | 100 |

(continued)

| Formulation 23 | Lip Color |
|---|---|
| Component | blending proportions (g/kg) |
| (7) Glyceryl behenate/eicosadioate | 20 |
| (8) Silica dimethyl silylate | 10 |
| (9) Propylparaben | 1 |
| (10) Hydrogenated polyisobutene | 350 |
| (11) Silicone-treated iron oxide pigment B | 10 |
| (12) Red No. 202 | 4 |

(Manufacturing Method)

(1) to (12) were heated and mixed until becoming uniform. After defoaming, a mixture was loaded into an appropriate container and then slowly cooled to room temperature, thereby obtaining an intended lip color. The obtained lip color exhibited a bright red unique tone.

| Formulation 24 | Lip Gloss |
|---|---|
| Component | blending proportions (g/kg) |
| (1) Dextrin palmitate | 100 |
| (2) Diisostearyl malate | 450 |
| (3) Liquid paraffin | Residue |
| (4) Preservative | 1 |
| (5) Antioxidant | 1 |
| (6) Iron oxide pigment A | 5 |
| (7) Red No. 202 | 1 |

(Manufacturing Method)

(1) to (5) were heated at 85°C and uniformly dissolved, and (6) and (7) were added thereto and uniformly dispersed. A dispersion was loaded into a container at a high temperature and rapidly cooled to room temperature, thereby obtaining an intended lip gloss. The obtained lip gloss exhibited a bright red unique tone.

A use for washing faces, removing dirt on bodies, or toothbrushing is also an aspect of the use of the cosmetic composition of the present invention.

| Formulation 25 | Body Shampoo |
|---|---|
| Component | blending proportions (g/kg) |
| (1) Lauric acid | 115 |
| (2) Myristic acid | 77 |
| (3) Palmitic acid | 48 |
| (4) Potassium hydroxide (purity: 480 g/kg) | 122 |
| (5) Lauryl hydroxysulfobetaine | 100 |
| (6) Cocamide monoethanolamine | 10 |
| (7) Ethylene glycol distearate | 20 |
| (8) Purified water | Residue |
| (9) EDTA-4Na | 1 |
| (10) Iron oxide pigment B | 10 |
| (11) Talc | 4 |

(Manufacturing Method)

(1) to (3) were heated from 70°C to 80°C and dissolved. Next, (4) was slowly added thereto, and saponification was performed. At a point in time where the saponification was ended, the remaining (5) to (9) were added thereto and stirred until becoming uniform. The components were cooled to 40°C, (10) and (11) mixed and pulverized were added thereto, and the components were cooled to room temperature while being stirred and mixed, thereby obtaining an intended body shampoo. The obtained body shampoo exhibited a brown vivid color.

| Formulation 26 Facial Cleansing Foam | |
| --- | --- |
| Component | blending proportions (g/kg) |
| (1) Stearic acid | 170 |
| (2) Myristic acid | 70 |
| (3) Lauric acid | 30 |
| (4) Potassium hydroxide (purity: 480 g/kg) | 120 |
| (5) PEG-60 glyceryl diisostearate | 30 |
| (6) Glyceryl stearate | 20 |
| (7) Sorbitol | 80 |
| (8) PEG 1500 | 100 |
| (9) Cocamidopropyl betaine | 50 |
| (10) Glycerin | 180 |
| (11) EDTA-4Na | 1 |
| (12) Purified water | Residue |
| (13) Pentylene glycol | 15 |
| (14) Iron oxide pigment C | 3 |
| (15) Talc | 6 |

(Manufacturing Method)

(1) to (3) were heated from 70°C to 80°C and dissolved. Next, (4) was slowly added thereto, and saponification was performed. At a point in time where the saponification was ended, the remaining (5) to (13) were added thereto and stirred until becoming uniform. The components were cooled to 40°C, (14) and (15) mixed and pulverized were added thereto, and the components were cooled to room temperature while being stirred and mixed, thereby obtaining an intended facial cleansing foam. The obtained facial cleansing foam exhibited a brown vivid color.

| Formulation 27 Facial Cleansing Powder | |
| --- | --- |
| Component | blending proportions (g/kg) |
| (1) Iron oxide pigment A | 3 |
| (2) Talc | 200 |
| (3) Lauric acid | 50 |
| (4) Mannitol | Residue |
| (5) Potassium myristate | 300 |
| (6) Glucose | 100 |
| (7) Potassium myristoyl glutamate | 20 |
| (8) Sodium methyl cocoyl taurate | 30 |
| (9) Betaine | 10 |
| (10) TANAKURA CLAY® | 50 |
| (11) Guar hydroxypropyltrimonium chloride | 5 |
| (12) Pentylene glycol | 10 |

(Manufacturing Method)

(1) to (12) were uniformly mixed with a Henschel mixer, thereby obtaining an intended facial cleansing powder. The obtained facial cleansing powder exhibited a brown vivid color.

| Formulation 28 | Solid Soap | |
| --- | --- | --- |
| Component | | blending proportions (g/kg) |
| (1) Fatty acid alkali metal salt | | 945 |
| | (lauric acid: 380 g/kg, myristic acid: 370 g/kg, palmitic acid: 150 g/kg, | |

(continued)

| Formulation 28 | Solid Soap | |
|---|---|---|
| Component | | blending proportions (g/kg) |
| stearic acid: 100 g/kg) | | |
| (potassium:sodium = 1:5) | | |
| (2) Cocamidopropyl betaine | | 30 |
| (3) Glycerin | | 20 |
| (4) Iron oxide pigment A | | 5 |

(Manufacturing Method)

(1) was heated and dissolved, (2) to (4) were added thereto and mixed therewith, and, furthermore, the components were homogenized with a triple roll. Next, the obtained mixture was kneaded and compressed by pressurization to be extruded in a rod shape with an extruder and molded with a die, thereby obtaining an intended solid soap. The obtained solid soap exhibited a brown vivid color.

| Formulation 29 | Powdered Bath Salt | |
|---|---|---|
| Component | | blending proportions (g/kg) |
| (1) Sodium sulfate | | 860 |
| (2) Sodium hydrogen carbonate | | Residue |
| (3) Monosodium glutamate | | 20 |
| (4) Silica (SUNSPHERE® H-52 manufactured by AGC Si-Tech Co., Ltd.) | | 10 |
| (5) Iron oxide pigment A | | 5 |
| (6) Vanillyl butyl ether | | 2 |
| (7) Capsicum extract | | 1 |

(Manufacturing Method)

(1) to (7) were uniformly mixed with a Henschel mixer, thereby obtaining an intended powdered bath salt. In the case of using the obtained powdered bath salt, bath water exhibited a brown vivid color.

| Formulation 30 | Peel-Off Pack | |
|---|---|---|
| Component | | blending proportions (g/kg) |
| (1) PEG 1500 | | 80 |
| (2) PEG/PPG-25/30 copolymer | | 60 |
| (3) Xanthan gum | | 2 |
| (4) Sodium citrate | | 3 |
| (5) Citric acid | | 1 |
| (6) Purified water | | Residue |
| (7) Polyvinyl alcohol | | 100 |
| (8) Polysorbate 80 | | 2 |
| (9) Silica (SUNSPHERE® H-51 manufactured by AGC Si-Tech Co., Ltd.) | | 40 |
| (10) Talc | | 80 |
| (11) Iron oxide pigment C | | 10 |
| (12) Alkyl acrylate copolymer | | emulsion 50 |
| (13) Methylparaben | | 2 |
| (14) Ethanol | | 80 |

(Manufacturing Method)

(9) to (11) mixed and pulverized were added to and uniformly dispersed in (1) to (8) heated and dissolved and then cooled at 40°C. Next, (12) to (14) were added thereto, stirred and cooled to room temperature, thereby preparing an intended peel-off pack. The obtained peel-off pack exhibited a unique vivid color.

| Formulation 31 | Toothpaste |
| Component | blending proportions (g/kg) |
| (1) Calcium carbonate | 290 |
| (2) Iron oxide pigment A | 5 |
| (3) Red No. 202 | 5 |
| (4) Sorbitol liquid | 35 |
| (5) Glycerin | 35 |
| (6) Guar gum | 30 |
| (7) Distilled water | 600 |

(Manufacturing Method)

(1) to (3) were weighed, added to (7), and stirred for five minutes using ROBOMICS fMODEL manufactured by Tokushu Kiki. Furthermore, (4) to (6) were added thereto under stirring, and the stirring was stopped when the viscosity of a mixture became large. The obtained toothpaste exhibited a vivid color and had both a viscosity high enough to put the toothpaste into tubes and flowability high enough for abrasives to enter gaps between teeth.

**Claims**

1. An iron oxide pigment for a cosmetic composition, wherein the iron oxide has a spinel structure or has a spinel structure and a corundum structure, and wherein a content of a Fe element is 680 g/kg or more and 702 g/kg or less, a FeO content is 30 g/kg or less, a $Fe_2O_3$ content is 939 g/kg or more, and a total content of FeO and $Fe_2O_3$ is 968 g/kg or more.

2. The iron oxide pigment according to Claim 1, wherein Pb is 10 mg/kg or less, As is 3 mg/kg or less, Hg is 1 mg/kg or less, Cd is 1 mg/kg or less, Zn is 100 mg/kg or less, Ba is 50 mg/kg or less, Cr is 100 mg/kg or less, Cu is 50 mg/kg or less, and Ni is 200 mg/kg or less.

3. The iron oxide pigment according to Claim 1 or 2, wherein, when an integrated intensity of diffraction lines of a (311) plane of a spinel-structured iron oxide appearing at a diffraction angle in a range of 35.10° or more and 36.10° or less in X-ray diffraction measurement is regarded as 100.00, an integrated intensity of diffraction lines of a (104) plane of a corundum-structured iron oxide appearing at a diffraction angle in a range of 32.60° or more and 33.60° or less is 10.0 or less.

4. The iron oxide pigment according to any one of Claims 1 to 3, wherein an average short axis length of primary particles is 50 nm or more and 500 nm or less.

5. The iron oxide pigment according to any one of Claims 1 to 4, wherein a BET specific surface area is 2.0 m²/g or more and 25.0 m²/g or less, a bulk density is 70 g/mL or less, and a residue after the iron oxide pigment is passed through a sieve with a mesh size of 45 μm is 1.0 g/kg or less.

6. The iron oxide pigment according to any one of Claims 1 to 5, wherein oil absorption is 20 g/100 g or more and 50 g/100 g or less.

7. A cosmetic composition comprising the iron oxide pigment according to any one of Claims 1 to 6.

8. The cosmetic composition according to Claim 7 that is a skin cosmetic.

9. The cosmetic composition according to Claim 7 or 8 that is a liquid cosmetic.

**10.** The cosmetic composition according to Claim 7 or 8 that is a solid cosmetic.

**11.** The cosmetic composition according to Claim 7 or 8 that is a gel cosmetic.

**12.** The cosmetic composition according to any one of Claims 7 to 11 that is used to produce dark color.

# Fig. 1

# Fig. 2

# Fig. 3

# Fig. 4

## INTERNATIONAL SEARCH REPORT

| International application No. |
| --- |
| **PCT/JP2022/014359** |

**A. CLASSIFICATION OF SUBJECT MATTER**

*A61K 8/19*(2006.01)i; *A61Q 1/02*(2006.01)i
FI:    A61K8/19; A61Q1/02

According to International Patent Classification (IPC) or to both national classification and IPC

**B. FIELDS SEARCHED**

Minimum documentation searched (classification system followed by classification symbols)

A61K8/19; A61Q1/02

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | JP 2016-763 A (TITAN KOGYO KABUSHIKI KAISHA) 07 January 2016 (2016-01-07) claims 1, 4, paragraphs [0008]-[0013] | 1-12 |
| A | JP 2010-83727 A (TITAN KOGYO KABUSHIKI KAISHA) 15 April 2010 (2010-04-15) entire text | 1-12 |
| A | US 4618375 A (BASF CORPORATION) 21 October 1986 (1986-10-21) entire text | 1-12 |
| A | JP 2012-240919 A (DAITO KASEI KOGYO KABUSHIKI KAISHA) 10 December 2012 (2012-12-10) entire text | 1-12 |
| A | JP 2020-26429 A (AMOREPACIFIC CORPORATION) 20 February 2020 (2020-02-20) entire text | 1-12 |

☐ Further documents are listed in the continuation of Box C.     ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **24 May 2022** | **07 June 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

INTERNATIONAL SEARCH REPORT
Information on patent family members

International application No.

**PCT/JP2022/014359**

| Patent document cited in search report | | | Publication date (day/month/year) | Patent family member(s) | Publication date (day/month/year) |
|---|---|---|---|---|---|
| JP | 2016-763 | A | 07 January 2016 | US 2015/0361242 A1 claims 1, 4, paragraphs [0008]-[0014] EP 2954885 A1 | |
| JP | 2010-83727 | A | 15 April 2010 | (Family: none) | |
| US | 4618375 | A | 21 October 1986 | DE 3534477 A1 | |
| JP | 2012-240919 | A | 10 December 2012 | (Family: none) | |
| JP | 2020-26429 | A | 20 February 2020 | KR 10-2020-0017820 A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014101298 A
- JP H02145506 A
- JP H11092148 A
- JP 2000336496 A